Europäisches Patentamt

European Patent Office

Office européen des brevets

(19)

(11) Veröffentlichungsnummer : **0 051 748
B1**

(12) EUROPÄISCHE PATENTSCHRIFT

(45) Veröffentlichungstag der Patentschrift :
30.01.85

(51) Int. Cl.⁴ : **G 01 N 33/80**

(21) Anmeldenummer : 81108009.2

(22) Anmeldetag : 07.10.81

(54) **Blutgruppenidentitätskarte, insbesondere für den Bed side-Test.**

(30) Priorität : 06.11.80 DEU 8029596

(43) Veröffentlichungstag der Anmeldung :
19.05.82 Patentblatt 82/20

(45) Bekanntmachung des Hinweises auf die Patenterteilung : 30.01.85 Patentblatt 85/05

(84) Benannte Vertragsstaaten :
AT BE CH DE FR GB IT LI LU NL SE

(56) Entgegenhaltungen :
DE-A- 2 921 136
DE-B- 1 005 759
US-A- 3 502 437

(73) Patentinhaber : **Biotest-Serum-Institut GmbH
Flughafenstrasse 4
D-6000 Frankfurt-Niederrad (DE)**

(72) Erfinder : **Uthemann, Horst, Dr. Dipl.-Chem.
Sachsenhäuser Landwehrweg 66
D-6000 Frankfurt 70 (DE)**
Erfinder : **Weber, Maria-Theresia
Elisabethstrasse 67
D-6070 Langen (DE)**

(74) Vertreter : **Beil, Walter, Dr. et al
BEIL, WOLFF & BEIL Rechtsanwälte Adelonstrasse
58
D-6230 Frankfurt am Main 80 (DE)**

**Beschreibung**

Die Erfindung betrifft eine Blutgruppenidentitätskarte, die insbesondere ihre Anwendung im Bed side-Test findet.

Unter Bed side-Test versteht man die Kontrolle der Identität der Blutgruppen des Empfängers und des Spenders, die direkt beim Patienten unmittelbar vor einer Bluttransfusion, was am häufigsten am Bett des Patienten geschieht, durchgeführt wird, um Verwechselungen jeglicher Art auszuschließen.

Gemäß den Richtlinien zur Blutgruppenbestimmung und Bluttransfusion der Bundesärztekammer vom 1.2.1979 (Deutsches Ärzteblatt, Heft 5 vom 1. Februar 1979) ist vor Transfusionen der ABO-Identitätstest am Krankenbett (Bed side-Test) vom Arzt oder unter seiner unmittelbaren Aufsicht durchzuführen (Deutsches Ärzteblatt, Heft 5, 1. Februar 1979, Seite 288, Absatzt 3.5.2).

Mit Blutidentitätskarten, auch Bed side-Karten genannt, lassen sich diese ABO-Identitätstests am Krankenbett auf einfache Weise durchführen.

So ist beispielsweise die sogenannte Eldon-Karte bekannt, die aus einer mit einer Zelluloseschicht überzogenen Pappe besteht, auf die ein Gemisch aus Dextran und den entsprechenden Antiseren auf Testfelder bzw. Versuchsabschnitte aufgetrocknet ist (Knud Eldon, Kopenhagen (1955) « Gleichzeitige ABO- und Rh-Blutgruppenbestimmung auf Karten im Laboratorium oder am Krankenbett », Broschüre der Nordisk Insulinlaboratorium, Gentoffe, Dänemark, 1-23 = Eldon, K. (1955), Simultaneous ABO and Rh groupings on cards in the laboratory or at the bed side. Danish Med. Bull. 2, 33-40 ;

Dissertationsarbeit von Anneliese Bär (1967) « Die Beeinflussung der Reaktionen inkompletter Rhesus-Antikörper durch Kolloide », Frankfurt). Die Zumischung von Dextran ist dort deshalb erforderlich, weil getrocknetes reines Serum nicht auf der dort verwendeten Unterlage haftet, sondern sich abschält, während Dextran zusammen mit den Serum auf der Unterlage fest haftet.

Ferner war bei den dort verwendeten Anti-D (Rh$_o$) Seren eine Verdünnung mit dem Konglutininsubstitut Dextran erforderlich, um beim teilweisen Trocknen einer Mischung aus Serum und Blutkörperchen die sogenannte Geldrollenbildung zu vermeiden, die in vielen Fällen Agglutinaten ähnlich sieht (Pseudoagglutination). Eine Verdünnung mit Dextran diente dabei außerdem der Förderung und Beschleunigung von Agglutinationsprozessen.

Dieses Dextran-Antiserum-Gemisch kann jedoch nicht direkt mit dem zu testenden Blut vermischt werden, sondern muß erst mit Wasser angelöst werden. Es ist deshalb bei jedem Test erforderlich, zunächst auf die Testfelder einen Tropfen Wasser aufzubringen. Dieser Tropfen Wasser muß sehr genau dosiert sein, wofür außer einer Pipette auch besondere Geschicklichkeit desjenigen erforderlich ist, der den Test durchführt. Das Erfordernis von Pipette, Wasser (was beispielsweise an einem Unfallort häufig nicht zur Verfügung steht), Geschicklichkeit des Ausführenden und der Zeitverlust durch das Auflösen stellen einen Nachteil dieser Testkarte dar.

Aus GM 79 04 755 ist eine Blutgruppenidentitätskarte bekannt, die aus einer Plastikplatte besteht, in die Vertiefungen eingepreßt sind, die mit Antiseren in flüssiger Form gefüllt und mit einer Plastikfolie verschlossen sind. Zur Durchführung der Tests mit dieser Karte muß die Plastikfolie mittels einer Kanüle oder Injektionsnadel, die das zu testende Blut enthält, durchstochen werden.

Damit erspart man sich zwar das Anlösen des Testserums. Da die Plastikfolie jedoch nur mit einer Spritze oder Kanüle durchstochen werden kann, besitzt diese Karte den Nachteil, daß zur Durchführung des Tests prinzipiell beim Patienten Venenblut entnommen werden muß, weil man mit einer Spritze kein Kapillarblut aus Fingerbeere oder Ohrläppchen entnehmen kann. Dies ist wesentlich aufwendiger als mit einer sterilen Lanzette Blut aus Fingerbeere oder Ohrläppchen zu entnehmen und aufzubringen, wie es bei der Eldon-Karte möglich ist.

Die beschriebene Karte besitzt ferner den Nachteil, daß flüssiges Antiserum im Gegensatz zu angetrocknetem Serum sich zum Zwecke der Dokumentation nicht fixieren läßt. Dies ist insofern besonders nachteilig, als die Richtlinien zur Blutgruppenbestimmung vorschreiben, daß eine Dokumentation möglich sein muß.

Aufgabe der Erfindung war es somit, eine Blutgruppenidentitätskarte, insbesondere für den Bed side-Test, zu entwickeln, auf deren Antiseren-Bereiche Kapillarblut aufgebracht werden kann und deren Reaktionsbild sich fixieren und konservieren läßt, also eine Karte, die mit angetrockneten Antiseren arbeitet, bei der jedoch auf das Auflösen der angetrockneten Antiseren mit Wasser verzichtet werden kann, d. h. bei der nach dem Aufbringen von Patienten- bzw. Transfusionsblut und Verrühren gleichzeitig ein rasches Auflösen der Antiseren erfolgt.

Diese Aufgabe wird erfindungsgemäß dadurch gelöst, daß die Blutgruppenidentitätskarte aus einer Polyesterfolie besteht, die mindestens auf einer Seite mit einer gleichmäßigen, gegenüber Antiseren haftfähigen Pigmentlackmattierungsschicht versehen ist, die auf ihrer Oberfläche auf räumlich voneinander getrennten Feldern festhaftende trockene Schichten aus im wesentlichen reinen Antiseren aufweist.

Es wurde überraschenderweise gefunden, daß auf einer mit Pigmentlackmattierung beschichteten Polyesterfolie, wie sie als Zeichenfolie auf dem Markt ist, Antiseren ohne jegliche haftvermittelnden Zusätze ausgezeichnet haften. Dadurch entfällt ein Haftmittel, wie es bei der Eldon-Karte erforderlich ist, und zugebenes Blut löst beim Verrühren die angetrockneten Antiseren auf, so daß auf das Auflösen mit Wasser verzichtet werden kann und der Test sehr leicht und schnell durchführbar ist. Das Reaktionsbild kann durch Übersprühen mit einem Lack, beispielsweise Zytolack zum Zwecke der Dokumentation leicht fixiert werden.

Durch den Einsatz von IgG der Spezifität Anti-D anstelle des bisher von Eldon verwendeten Anti-D-Serums ist es außerdem möglich, auch bei der angetrockneten Anti-D-Schicht auf den Zusatz von Dextran zu verzichten.

Chemisch modifizierte IgG-Moleküle, bei denen Disulfidbrücken in der Hinge-Region teilweise reduktiv aufgespalten und alkyliert worden sind, zeichnen sich durch hohe Flexibilität aus, weshalb bei Antiseren auf der Basis der modifizierten Moleküle auf den üblichen Zusatz von Supplement (z. B. Albumin, Dextran, Gelatine, AB-Serum u. a.) verzichtet werden kann. Die chemische Modifizierung überführt inkomplette Antikörper in Agglutinine, die ähnliche Eigenschaften wie ABO-Testseren haben. So werden mit modifiziertem IgG der Spezifität Anti-D D(Rh$_o$)-positive Erythrozyten in Kochsalzlösung oder in verträglichem Serum bzw. Plasma agglutiniert. In Pirofsky B. und Cordova M.S. « Bivalent nature of incomplete anti-D (Rh$_0$) » Nature 197, S. 392-393 (1963) ; Roman D., Tilley C. A., Crookstone N. C., Falk R. E. and Dorington K. J. « Conversion of incomplete antibodies to direct agglutinins by mild reduction. Evidence for segmental flexibility within the F$_c$ fragment of immunoglobuline G » Proc. Natl. Akad. Sci. USA, 74, S. 2531-2535, (1977) wird dieses Anti-D-Reagens beschrieben. Bisher wurden der Objektträger-Schnelltest, der Objektträger-Inkubationstest und der Röhrchenzentrifugiertest als Anwendungsmöglichkeiten vorgeschlagen.

Überraschenderweise wurde gefunden, daß das Anti-D-Reagens, auf Polyesterfolie angetrocknet und ohne vorher mit Wasser aufgelöst werden zu müssen, beim Verrühren mit D(Rh$_o$)-positivem Blut nach kurzer Reaktionszeit (ca. 1 Minute) in ihrer Stärke dem ABO-System vergleichbare Agglutinationsbilder liefert. Weil weder das Reagens selbst Supplemente enthält, noch für das Antrocknen auf der ausgewählten Polyesterfolie hochpolymere, eine Haftung vermittelnde Zusätze erforderlich sind, kann anders als bei der Eldon-Karte auf eine Kontrolle verzichtet werden. Es ist nicht zu erwarten und wurde bisher auch nicht beschrieben, daß das Anti-D-Reagens mit seiner niedriger Proteinkonzentration, die der Konzentration von normalem humanen Serum entspricht, zu falsch positiven Reaktionen mit IgG- und/oder komplementbeladenen Erythrozyten bei Autoimmunerkrankungen oder der Erythroblastose des Neugeborenen führt, die aber häufig mit inkompletten Anti-Rh-Seren, die Rinderalbumin oder andere hochmolekulare Substanzen als Verstärkermedium enthalten, beobachtet werden.

Ein besonders geeignetes Folienmaterial ist eine Polyesterfolie, die im Extrudierverfahren hergestellt und durch biaxiale Streckung und Hitzestabilisierung dimensionsstabil gemach wurde und auf beiden Seiten mit einer gleichmäßigen Pigmentlackmattierung beschichtet ist.

Eine besonders zweckmäßige Folie besitzt folgende mechanische und physikalische Eigenschaften :

Zugfestigkeit 1 800 kg/cm$^2$, Streckgrenze 980 kg/cm$^2$,

thermische Ausdehnung 0,027 mm/m/$^o$C,

Feuchtigkeitsausdehnung 0,01 mm/m/% RF,

Wärmefestigkeit 150 $^o$C

Eine derart besonders zweckmäßige Folie ist unter der Bezeichnung Zeichenfolie Safir PL Opak-weiß im Handel erhältlich.

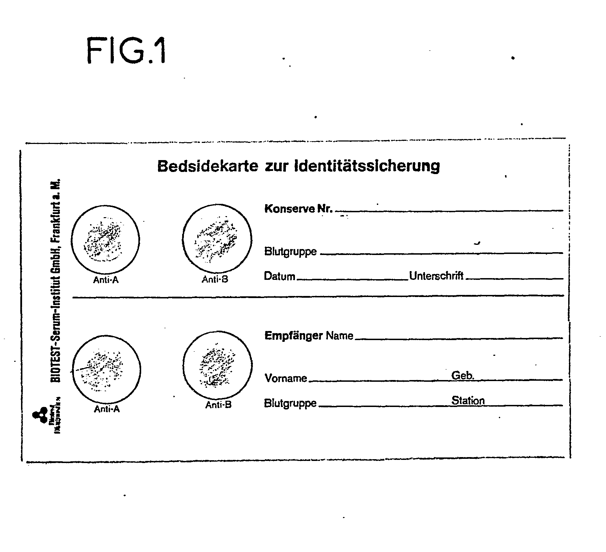

Für Zwecke, wo im Bed side-Test nur die ABO-Identität zu prüfen ist, besitzt eine Ausführungsform der erfindungsgemäßen Karte, wie sie in Fig. 1 wiedergegeben ist, vier räumlich voneinander getrennte Felder, von denen je zwei mit Antiserum A und Antiserum B beschichtet sind.

Die Felder sind mit Anti-A bzw. Anti-B gekennzeichnet, und die Antiseren-Schichten können zusätzlich, um Verwechselungen zu vermeiden, unterschiedlich eingefärbt sein.

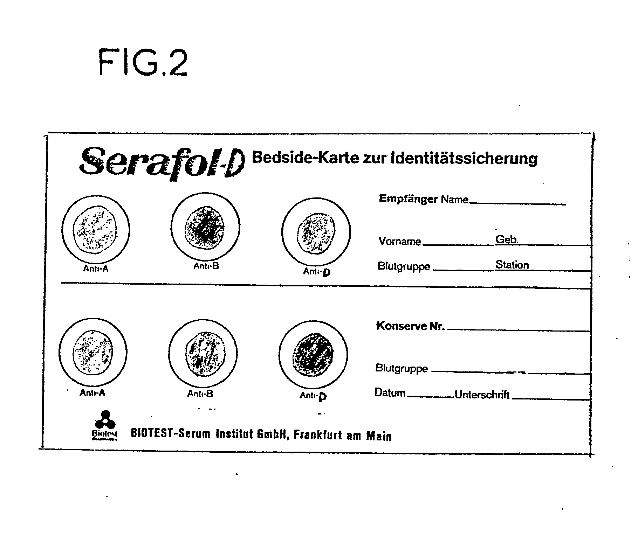

Für Zwecke, wo im Bed side-Test auch der Rh-Faktor bestimmt werden soll, besitzt eine Ausführungsform der erfindungsgemäßen Karte, wie sie in Fig. 2 wiedergegeben ist, sechs räumlich voneinander getrennte Felder, von denen je zwei mit Antiserum A, Antiserum B und Anti-D beschichtet sind.

Die einzelnen Felder sind so angeordnet, daß noch genügend Raum für eine Beschriftung der Folie vorhanden ist. Bei der bevorzugten beidseitigen pigmentlackmattierten Folie kann auch auf der Rückseite der Folie eine Beschriftung durchgeführt werden.

**Ansprüche**

1. Blutgruppenidentitätskarte, insbesondere für den Bed side-Test, dadurch gekennzeichnet, daß sie aus einer Polyesterfolie besteht, die mindestens auf einer Seite mit einer gleichmäßigen, gegenüber Antiseren haftfähigen Pigmentlackmattierungsschicht versehen ist, die auf ihrer Oberfläche auf räumlich voneinander getrennten Feldern festhaftende trockene Schichten aus im wesentlichen reinen Antiseren aufweist.

2. Karte nach Anspruch 1, dadurch gekennzeichnet, daß die Schichten aus Antiserum A und Antiserum B bestehen.

3. Karte nach Anspruch 2, dadurch gekennzeichnet, daß eine weitere Schicht aus IgG der Spezifität Anti-D besteht.

4. Karte nach Anspruch 3, dadurch gekennzeichnet, daß die Karte sechs Felder aufweist, von denen je zwei mit Antiserum A, zwei mit Antiserum B und zwei mit Antiserum D beschichtet sind.

5. Karte nach einem der vorstehenden Ansprüche, dadurch gekennzeichnet, daß die Schichten unterschiedlicher Antiseren mit unterschiedlicher Färbung versehen sind.

6. Karte nach einem der vorstehenden Ansprüche, dadurch gekennzeichnet, daß die Polyesterfolie auf beiden Seiten mit einer Pigmentlackmattierungsschicht versehen ist.

## Claims

1. Blood group identification card, particularly for the bed side test, characterized in that, it consists of a sheet of polyester which has at least on one side a uniform pigment-lacquer dulling layer that has the capacity to adhere to antisera, which layer has on its surface in separated fields tightly adhering dry layers of essentially pure antisera.

2. Card according to claim 1, characterized in that the layers consist of antisera A and antisera B.

3. Card according to claim 2, characterized in that a further layer consists of IgG of the specificity anti-D.

4. Card according to claim 3, characterized in that the card has six fields, two of each being coated with antiserum A, two with antiserum B and two with antiserum D.

5. Card according to one of the precedent claims, characterized in that the layers of different antisera are provided with different colouring.

6. Card according to one of the precedent claims, characterized in that the sheet of polyester has on both sides a pigment-lacquer dulling layer.

## Revendications

1. Carte d'identité de groupes sanguins, en particulier pour le Bed side-test, caractérisée en ce qu'elle est constituée d'une feuille de polyester qui est munie sur au moins une de ses faces d'une couche régulière d'enduit à base de pigment matant favorisant l'adhérence d'antisérums, qui, sur sa surface supérieur présente des plages séparées l'une de l'autre de couches sèches adhérant solidement, constituées pour l'essentiel d'antisérums purs.

2. Carte selon la revendication 1, caractérisée en ce que les couches sont constituées d'antisérum A et d'antisérum ·B.

3. Carte selon la revendication 2, caractérisée en ce qu'une autre couche est constituée de IgG de spécificité anti-D.

4. Carte selon la revendication 3, caractérisée en ce que la carte présente six plages, dont chaque fois deux sont enduites avec l'antisérum A, deux avec l'antisérum B et deux avec l'antisérum D.

5. Carte selon l'une des revendications précédentes, caractérisée en ce que les plages de différents sérums sont colorées différemment.

6. Carte selon l'une des revendications précédentes, caractérisée en ce que la feuille de polyester est munie sur ses deux faces d'une couche d'enduit à base de pigment matant.

# FIG.1

**Bedsidekarte zur Identitätssicherung**

BIOTEST-Serum-Institut GmbH, Frankfurt a. M.

Anti-A     Anti-B

Konserve Nr. _____

Blutgruppe _____

Datum_____ Unterschrift _____

Anti-A     Anti-B

**Empfänger** Name_____

Vorname_____ Geb._____

Blutgruppe_____ Station_____

# FIG.2